# EUROPEAN PATENT APPLICATION

(11) **EP 4 663 018 A1**
(43) Date of publication of application: **17.12.2025**
(21) Application number: 24753274.0
(22) Date of filing: 02.02.2024
(51) Int. Cl.: A01N 37/36, A01N 25/02, A01N 25/30, A01N 59/00, A01P 3/00, A61L 2/14, A61L 2/18

(54) **PLASMA-IRRADIATED AQUEOUS SOLUTION AND PRODUCTION METHOD THEREOF, PLASMA-IRRADIATED AQUEOUS SOLUTION GENERATION DEVICE, AND STERILIZATION METHOD**

(30) Priority: 06.02.2023 JP 2023016083
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: HASHIZUME, Hiroshi, Nagoya-shi, Aichi 464-8601 (JP); MIZUNO, Masaaki, Nagoya-shi, Aichi 464-8601 (JP); HORI, Masaru, Nagoya-shi, Aichi 464-8601 (JP)
(74) Representative: Kramer Barske Schmidtchen Patentanwälte PartG mbB
(86) International application number: PCT/JP2024/003570
(87) International publication number: WO 2024/166831

(57) **Abstract**

An aqueous solution containing at least one selected from the group consisting of sodium lactate, potassium lactate, calcium lactate, and lactic acid, and a surfactant is irradiated with plasma to produce a plasma-irradiated aqueous solution. The surfactant can be at least one selected from a nonionic surfactant, a cationic surfactant, an anionic surfactant, and an amphoteric surfactant. The aqueous solution preferably contains at least sodium lactate. The concentration of the surfactant in the aqueous solution can be 2% by volume or less. By bringing the plasma-irradiated aqueous solution into contact with true fungi or fungi, the true fungi or fungi are sterilized.

## Description

### CROSS-REFERENCE TO RELATED APPLICATIONS

The present application is based on Japanese Application No. 2023-016083 filed on February 6, 2023, the contents of which are incorporated herein by reference.

### TECHNICAL FIELD

The present disclosure relates to a plasma-irradiated aqueous solution, a method for producing the same, a plasma-irradiated aqueous solution generation device, and a sterilization method.

### BACKGROUND ART

Plasma technology is applied in the fields of electricity, chemistry, and materials. For example, in the field of sterilization of microorganisms, a technique for sterilizing microorganisms by directly irradiating them with plasma is known.

Further, as described in Patent Literature 1, a technique has also been proposed for sterilizing true fungi or fungi by indirect treatment using a plasma-irradiated aqueous solution obtained by irradiating a culture solution with plasma, without direct plasma irradiation.

### PRIOR ART LITERATURE

### Patent Literature

Patent Literature 1: JP 2016-150923 A

### SUMMARY OF INVENTION

### PROBLEMS TO BE SOLVED BY INVENTION

In a case of direct plasma irradiation, sterilization treatment can be performed only at a location where a plasma irradiation device is installed. In contrast, the plasma-irradiated aqueous solution of Patent Literature 1 is highly portable, allowing sterilization treatment to be performed at any location.

However, although the conventional plasma-irradiated aqueous solution exhibits a sterilization effect against true fungi or fungi, the sterilization effect is not so large and there is room for improvement.

The present disclosure has been made in view of such problems, and an object of the present disclosure is to provide a plasma-irradiated aqueous solution that exhibits a high sterilization effect against true fungi or fungi, as well as a sterilization method using the plasma-irradiated aqueous solution.

### MEANS FOR SOLVING PROBLEM

One aspect of the present disclosure is a plasma-irradiated aqueous solution obtained by irradiating an aqueous solution containing at least one selected from the group consisting of sodium lactate, potassium lactate, calcium lactate, and lactic acid, and a surfactant, with plasma.

Another aspect of the present disclosure is a method for producing a plasma-irradiated aqueous solution, including:
a preparation step of preparing an aqueous solution containing at least one selected from the group consisting of sodium lactate, potassium lactate, calcium lactate, and lactic acid, and a surfactant; and
a plasma irradiation step of irradiating the aqueous solution prepared in the preparation step with plasma to generate a plasma-irradiated aqueous solution.

Still another aspect of the present disclosure is a plasma-irradiated aqueous solution generation device having a plasma irradiation unit that irradiates an aqueous solution containing at least one selected from the group consisting of sodium lactate, potassium lactate, calcium lactate, and lactic acid, and a surfactant, with plasma to generate a plasma-irradiated aqueous solution.

Still another aspect of the present disclosure is a sterilization method in which the plasma-irradiated aqueous solution, a plasma-irradiated aqueous solution obtained by the method for producing a plasma-irradiated aqueous solution, or a plasma-irradiated aqueous solution generated by the plasma-irradiated aqueous solution generation device is brought into contact with true fungi or fungi to sterilize the true fungi or the fungi.

### EFFECTS OF INVENTION

Since the plasma-irradiated aqueous solution is obtained by irradiating an aqueous solution containing at least one selected from the group consisting of sodium lactate, potassium lactate, calcium lactate, and lactic acid (hereinafter, these may be collectively referred to as sodium lactate or the like.) and a surfactant with plasma, it can exhibit a higher sterilization effect against true fungi or fungi as compared to that obtained by plasma-irradiating an aqueous solution that contains sodium lactate or the like but does not contain a surfactant.

Since the method for producing a plasma-irradiated aqueous solution includes the above steps, the plasma-irradiated aqueous solution exhibiting a high sterilization effect against true fungi or fungi can be produced.

Since the plasma-irradiated aqueous solution generation device has the above configuration, it is possible to generate the plasma-irradiated aqueous solution exhibiting a high sterilization effect against true fungi or fungi.

In the sterilization method, the plasma-irradiated aqueous solution, a plasma-irradiated aqueous solution obtained by the method for producing a plasma-irradiated aqueous solution, or a plasma-irradiated aqueous solution generated by the plasma-irradiated aqueous solution generation device is brought into contact with true fungi or fungi. Therefore, the sterilization method can efficiently sterilize true fungi or fungi.

### BRIEF DESCRIPTION OF DRAWINGS

Fig. 1 is a diagram showing a schematic configuration of a plasma-irradiated aqueous solution generation device of Embodiment 3.
Fig. 2(a) is a cross-sectional view showing the configuration of a plasma generation unit in the plasma-irradiated aqueous solution generation device of Embodiment 3, and Fig. 2(b) is a partial cross-sectional view in a cross section perpendicular to a longitudinal direction of a plasma region.
Fig. 3 is a photograph showing the state of Penicillium digitatum spores after treatment with various solutions prepared in Experimental Example 1.
Fig. 4 is a diagram showing a relationship between treatment time (h) (horizontal axis) and a viable cell count of Penicillium digitatum spores (CFU/mL) (vertical axis) using various solutions prepared in Experimental Example 1.
Fig. 5 is a photograph showing the state of Penicillium digitatum spores after treatment with various solutions prepared in Experimental Example 2.
Fig. 6 is a diagram showing the relationship between the treatment time (h) (horizontal axis) and the viable cell count of Penicillium digitatum spores (CFU/mL) (vertical axis) using various solutions prepared in Experimental Example 2.
Fig. 7 is a diagram showing the relationship between the treatment time (h) (horizontal axis) and the viable cell count of Penicillium digitatum spores (CFU/mL) (vertical axis) using various solutions prepared in Experimental Example 3.
Fig. 8 is a diagram showing the relationship between the treatment time (h) (horizontal axis) and the viable cell count of Penicillium digitatum spores (CFU/mL) (vertical axis) using various solutions prepared in Experimental Example 4.
Fig. 9 is a photograph showing the state of Aspergillus flavus spores after treatment with various solutions prepared in Experimental Example 5.
Fig. 10 is a photograph showing the state of Aspergillus niger spores after treatment with various solutions prepared in Experimental Example 6.
Fig. 11 is a photograph showing the state of Aspergillus niger spores after treatment with various solutions prepared in Experimental Example 6.
Fig. 12 is a photograph showing the state of Glomerella acutata spores after treatment with various solutions prepared in Experimental Example 7.
Fig. 13 is a photograph showing the state of Trichophyton after treatment with various solutions prepared in Experimental Example 8.
Fig. 14 is a diagram showing the relationship between the treatment time (h) (horizontal axis) and the viable cell count of Trichophyton (cultured for 5 days) (CFU/mL) (vertical axis) using various solutions prepared in Experimental Example 8.
Fig. 15(a) shows a molecular structure of polyoxyethylene (20) Sorbitan Monolaurate (Tween20), and Fig. 15(b) shows a molecular structure of polyoxyethylene (20) Sorbitan Monooleate (Tween80).
Fig. 16 is a diagram showing the relationship between the treatment time (h) (horizontal axis) and the viable cell count of Penicillium digitatum spores (CFU/mL) (vertical axis) using various solutions prepared in Experimental Example 9.
Fig. 17 is a diagram showing the relationship between the treatment time (h) (horizontal axis) and the viable cell count of Penicillium digitatum spores (CFU/mL) (vertical axis) using various solutions prepared in Experimental Example 9.
Fig. 18 is a diagram showing the relationship between the treatment time (h) (horizontal axis) and the viable cell count of Penicillium digitatum spores (CFU/mL) (vertical axis) using various solutions prepared in Experimental Example 10.
Fig. 19 is a diagram showing the relationship between the treatment time (h) (horizontal axis) and the viable cell count of Penicillium digitatum spores (CFU/mL) (vertical axis) using various solutions prepared in Experimental Example 11.

### MODE FOR CARRYING OUT INVENTION

Hereinafter, the plasma-irradiated aqueous solution, the method for producing the same, the plasma-irradiated aqueous solution generation device, and the sterilization method of the present embodiment will be described in detail with reference to the drawings as appropriate. Note that, the plasma-irradiated aqueous solution, the method for producing the same, the plasma-irradiated aqueous solution generation device, and the sterilization method of the present embodiment are not limited by the following examples.

### (Embodiment 1)

A plasma-irradiated aqueous solution of Embodiment 1 will be described. The plasma-irradiated aqueous solution of the present embodiment is obtained by irradiating an aqueous solution containing a specific component with plasma. In other words, the plasma-irradiated aqueous solution of the present embodiment is in a state after the aqueous solution containing a specific component is irradiated with plasma.

Specifically, the aqueous solution containing a specific component before plasma irradiation contains at least one selected from the group consisting of sodium lactate, potassium lactate, calcium lactate, and lactic acid, and a surfactant. The aqueous solution preferably contains at least sodium lactate from a viewpoint of having a large sterilization effect against true fungi or fungi, being easily available, and the like. In other words, in this case, the aqueous solution may contain one kind of sodium lactate and a surfactant, or may contain sodium lactate, at least one selected from the group consisting of potassium lactate, calcium lactate, and lactic acid, and a surfactant.

The surfactant can be at least one selected from a nonionic surfactant, a cationic surfactant, an anionic surfactant, and an amphoteric surfactant. This configuration allows for a wider range of selection of the type and concentration of surfactant. Among these surfactants, a nonionic surfactant or an anionic surfactant is preferably contained from the viewpoint of having a large sterilization effect against true fungi or fungi, a wide range of selection of the type of surfactant, and ensuring the sterilization effect by plasma treatment.

Examples of the nonionic surfactant include sucrose fatty acid ester; polyethylene glycol fatty acid esters such as polyethylene glycol monooleate and polyethylene glycol dioleate; sorbitan fatty acid esters such as sorbitan sesquioleate, propylene glycol fatty acid esters such as proolein glycol monostearate; polyoxyethylene hydrogenated castor oil such as polyoxyethylene hydrogenated castor oil 60; polyoxyethylene sorbitan fatty acid esters such as polyoxyethylene (20) Sorbitan Monolaurate (Tween20) and polyoxyethylene (20) Sorbitan Monooleate (Tween80); glycerin fatty acid esters such as glyceryl monostearate; polyoxyethylene glycerin fatty acid esters such as polyoxyethylene glycerin monostearate; polyoxyethylene alkyl ethers such as polyoxyethylene lauryl ether; block polymer type such as polyoxyethylene (160) polyoxypropylene (30) glycol; polyoxyethylene alkyl aryl ethers such as polyoxyethylene nonylphenyl ether; and nitrogen-containing types such as polyoxyethylene oleylamine and coconut oil diethanolamide. These can be used alone or in combination of two or more thereof.

Examples of the cationic surfactant include quaternary ammonium salts such as benzalkonium chloride. These can be used alone or in combination of two or more thereof.

Examples of the anionic surfactant include alkyl aryl sulfonates such as sodium alkylbenzene sulfonate (LAS); alkyl sulfates such as sodium lauryl sulfate; polyoxyethylene alkyl ether phosphates such as sodium polyoxyethylene cetyl ether phosphate; and bile acids such as ursodeoxycholic acid. These can be used alone or in combination of two or more thereof.

Examples of the amphoteric surfactant include alkyl polyaminoethylglycine hydrochloride such as alkyl diaminoethylglycine hydrochloride, lecithin such as purified soybean lecithin and purified egg yolk lecithin. These can be used alone or in combination of two or more thereof.

The surfactant is preferably used for pharmaceuticals. In this case, since it is used in medical and clinical settings, there are advantages that an amount of information on safety and handleability is abundant, the influence on the human body is small, and it is easy to use in medical and clinical settings. Note that, any of the surfactants specifically listed above can be used for pharmaceuticals.

The concentration of the surfactant in the aqueous solution can be 2% by volume or less. At or below this concentration, a concentration-dependent sterilization effect against true fungi or fungi can be ensured. Further, there is also an advantage that it can be diluted and used according to the object and application. The concentration of the surfactant in the aqueous solution can be preferably 0.005% by volume or more, more preferably 0.008% by volume or more, and still more preferably 0.01% by volume or more from the viewpoint of ensuring the sterilization effect against true fungi or fungi. Further, the concentration of the surfactant in the aqueous solution can be preferably 1.5% by volume or less, more preferably 1% by volume or less, still more preferably 0.5% by volume or less, and still more preferably 0.1% by volume or less from the viewpoint of improving handleability and the like. Note that, the concentration of the surfactant in the aqueous solution can be determined with reference to, for example, the concentration of the surfactant used in pharmaceuticals.

The concentration of sodium lactate or the like in the aqueous solution can be, for example, 0.5 g/L or more and 5 g/L or less. Specifically, the concentration of sodium lactate or the like in the aqueous solution can be, for example, 3.1 g/L (27.6 mM) or the like according to the composition of a commercially available sodium lactate Ringer's solution.

The plasma-irradiated aqueous solution of the present embodiment is activated for sterilization by irradiating the aqueous solution with plasma. Specifically, the plasma to be applied to the aqueous solution can be non-equilibrium atmospheric pressure plasma (low temperature atmospheric pressure plasma). Note that, the atmospheric pressure plasma referred to in the present disclosure refers to plasma generated not only under atmospheric pressure but also under a pressure within a range of 0.5 atm or more and 2.0 atm or less. Examples of gases used for plasma generation mainly include rare gases such as Ar, He, and Ne.

As a plasma irradiation device for irradiating the aqueous solution with plasma, devices described in JP 2009-87697 A, JP 2016-150923 A, and the like can be suitably used. The techniques of each of these publications can be incorporated in the present disclosure as necessary.

A plasma density can be, for example, 1×10¹⁴ cm⁻³ or more and 1×10¹⁷ cm⁻³ or less. Further, a plasma irradiation time can be, for example, 30 seconds or more and 30 minutes or less. Further, a plasma temperature can be, for example, 1000 K or more and 2500 K or less. Further, the number of times of plasma irradiation may be one or a plurality of times. Further, a plasma irradiation distance can be, for example, more than 0 mm and 30 mm or less.

Since the plasma-irradiated aqueous solution of the present embodiment is obtained by irradiating an aqueous solution containing sodium lactate or the like and a surfactant with plasma, it can exhibit a higher sterilization effect against true fungi or fungi as compared with that obtained by irradiating an aqueous solution that contains sodium lactate or the like but does not contain a surfactant with plasma.

The plasma-irradiated aqueous solution of the present embodiment has a sterilization activity against true fungi or fungi as described above. Therefore, the plasma-irradiated aqueous solution of the present embodiment can be suitably used as an aqueous solution for sterilization for sterilizing true fungi or fungi. Note that, in the present disclosure, the term fungi is used in a broad sense including true fungi.

Specific examples of true fungi include Penicillium digitatum, Aspergillus flavus, Aspergillus niger, Glomerella acutata, Fusarium, Magnaporthe oryzae, Gibberella fujikuroi, Trichophyton, and Candida. Examples of other fungi include Escherichia coli, Staphylococcus aureus, bacterial leaf blight virus, coronavirus, and stripe virus.

Further, the plasma-irradiated aqueous solution of the present embodiment can also be suitably used for sterilizing bacteria and viruses, which have a weaker microbial structure than true fungi or fungi.

The plasma-irradiated aqueous solution of the present embodiment can be used in various fields such as a food field, an agricultural field, a medical field, a pharmaceutical field, and a living field by utilizing the sterilization activity against true fungi or fungi. In the food field, it can be applied to, for example, antifungal measures during food production and distribution of vegetables, fruits, and other foods. In the agricultural field, it can be applied to, for example, sterilization treatment during crop cultivation, and antifungal measures for cultivated vegetables and fruits. In the medical field, it can be applied to, for example, wiping and cleaning of bedridden elderly people (prevention, improvement, and the like of senile vaginosis, scrotal mycosis, decubitus and the like), antiviral (COVID19, papilloma, etc.), anticancer, anti-aging (beauty), medical instrument cleaning equipment, and the like. In the living field, it can be applied to, for example, toilet cleaning, feces processing, and the like.

### (Embodiment 2)

A method for producing a plasma-irradiated aqueous solution of Embodiment 2 will be described. The method for producing a plasma-irradiated aqueous solution of the present embodiment includes a preparation step and a plasma irradiation step.

The preparation step is a step of preparing an aqueous solution containing at least one selected from the group consisting of sodium lactate, potassium lactate, calcium lactate, and lactic acid, and a surfactant. The aqueous solution can be prepared, for example, by mixing a predetermined amount of sodium lactate or the like and a predetermined amount of a surfactant in distilled water. At this time, the order of mixing sodium lactate or the like with the surfactant is not particularly limited. For example, the aqueous solution can also be prepared by preparing a stock solution of the aqueous solution and diluting the stock solution with distilled water. For example, the aqueous solution can also be prepared by mixing an aqueous solution that does not contain a surfactant but contains sodium lactate or the like with an aqueous solution that does not contain sodium lactate or the like but contains a surfactant. For example, the aqueous solution can also be prepared by adding a surfactant to an aqueous solution that does not contain a surfactant but contains sodium lactate or the like, and mixing them. Details of the aqueous solution are the same as those in Embodiment 1, and thus description thereof will be omitted.

The plasma irradiation step is a step of irradiating the aqueous solution prepared in the preparation step with plasma to generate a plasma-irradiated aqueous solution. A plasma irradiation device for irradiating the aqueous solution with plasma, plasma irradiation conditions, and the like are the same as those in Embodiment 1, and thus the description thereof will be omitted.

Since the method for producing a plasma-irradiated aqueous solution of the present embodiment includes the above steps, the plasma-irradiated aqueous solution of the present embodiment exhibiting a high sterilization effect against true fungi or fungi can be produced.

Other configurations and operational effects are the same as those described in the description of the plasma-irradiated aqueous solution of Embodiment 1, and it is also possible to appropriately refer to the description of other embodiments.

### (Embodiment 3)

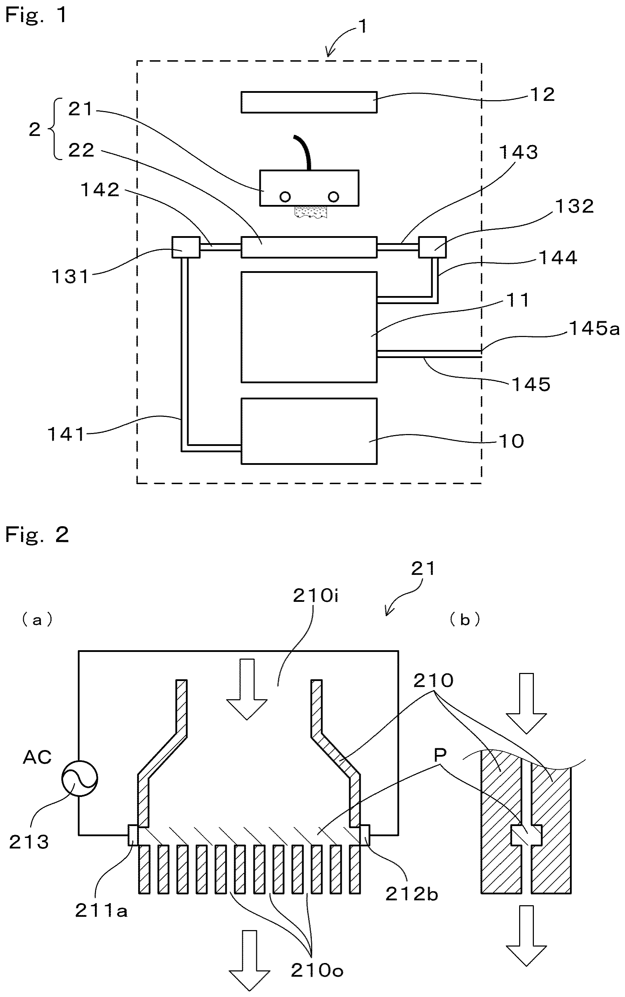

A plasma-irradiated aqueous solution generation device of Embodiment 3 will be described with reference to Figs. 1 and 2. As illustrated in Fig. 1, a plasma-irradiated aqueous solution generation device 1 of the present embodiment includes a plasma irradiation unit 2.

Specifically, the plasma-irradiated aqueous solution generation device 1 illustrated in Fig. 1 includes the plasma irradiation unit 2, an aqueous solution storage tank 10, a plasma-irradiated aqueous solution storage tank 11, a power supply unit 12, pumps 131 and 132, and flow channels 141, 142, 143, 144, and 145.

The plasma irradiation unit 2 irradiates an aqueous solution containing at least one selected from the group consisting of sodium lactate, potassium lactate, calcium lactate, and lactic acid and a surfactant with plasma to generate a plasma-irradiated aqueous solution. Fig. 1 shows an example in which the plasma irradiation unit 2 includes a plasma generation unit 21 and a reaction tank 22.

The plasma generation unit 21 is a part that generates plasma to be applied to the aqueous solution of a raw material, and details thereof will be described later. The reaction tank 22 is a water tank for storing the aqueous solution of the raw material or the plasma-irradiated aqueous solution generated by plasma irradiation. The plasma irradiation unit 2 is configured to enable plasma irradiation for a plasma irradiation time by setting the plasma irradiation time in advance.

The aqueous solution storage tank 10 is for storing the aqueous solution serving as a raw material before plasma irradiation. The plasma-irradiated aqueous solution storage tank 11 is for storing the plasma-irradiated aqueous solution generated by plasma irradiation. The power supply unit 12 is for supplying power to each unit. The power supply unit 12 includes a control unit (not illustrated) that controls each unit.

The pump 131 is for supplying the aqueous solution of the raw material from the aqueous solution storage tank 10 to the reaction tank 22 of the plasma irradiation unit 2. The pump 132 is for supplying the plasma-irradiated aqueous solution from the reaction tank 22 of the plasma irradiation unit 2 to the plasma-irradiated aqueous solution storage tank 11.

The flow channel 141 connects the aqueous solution storage tank 10 and the pump 131. The flow channel 142 connects the pump 131 and the reaction tank 22 of the plasma irradiation unit 2. The flow channel 143 connects the reaction tank 22 of the plasma irradiation unit 2 and the pump 132. The flow channel 144 connects the pump 132 and the plasma-irradiated aqueous solution storage tank 11. The flow channel 145 connects the plasma-irradiated aqueous solution storage tank 11 and an outlet port 145a for taking out the plasma-irradiated aqueous solution.

The control unit of the power supply unit 12 controls each unit as follows. First, the aqueous solution of the raw material is supplied from the aqueous solution storage tank 10 to the reaction tank 22 of the plasma irradiation unit 2. Next, the plasma irradiation unit 2 irradiates the aqueous solution in the reaction tank 22 with plasma to generate a plasma-irradiated aqueous solution. Next, the plasma-irradiated aqueous solution is supplied from the reaction tank 22 to the plasma-irradiated aqueous solution storage tank 11. Next, the plasma-irradiated aqueous solution storage tank 11 stores the plasma-irradiated aqueous solution. Then, the plasma-irradiated aqueous solution is dispensed by an arbitrary predetermined amount from the outlet port 145a.

In the plasma-irradiated aqueous solution generation device 1, specifically, the plasma generation unit 21 can be configured as follows, for example. As illustrated in Fig. 2, the plasma generation unit 21 includes a housing 210, an electrode 211a, an electrode 212b, and a voltage application unit 213.

A gas inlet port 210i is formed at one end of the housing 210, and a large number of gas outlet ports 210o are formed at the other end of the housing 210. The gas inlet port 210i is for introducing gases such as Ar for generating plasma. The gas inlet port 210i has a slit shape with the longitudinal direction being a left-right direction in Fig. 2(a). The gas inlet port 210i is designed to introduce gases in a direction that intersects a line connecting the electrode 211a and the electrode 212b. The gas outlet port 210o is for irradiating the outside of the housing 210 with plasma. The gas outlet port 210o can have a cylindrical shape or a slit shape. The gas outlet port 210o in the case of a cylindrical shape can be formed in a straight line along the longitudinal direction of the plasma region.

The electrode 211a and the electrode 212b are a counter electrode pair arranged to face each other. In the electrode 211a and the electrode 212b, a large number of recesses (hollows) are formed on each of opposing surfaces. The electrode 211a and the electrode 212b are disposed inside the housing 210 and near the gas outlet port 210o. The voltage application unit 213 applies an AC voltage between the electrode 211a and the electrode 211b.

The plasma generation unit 21 can eject plasma in a straight line by applying a voltage between the electrode 211a and the electrode 211b using a commercial AC voltage. Further, if the plasma generation units 21 that eject the plasma in a straight line are arranged in a row in the left-right direction in Fig. 2(b), the plasma can be ejected in a planar manner over a certain rectangular region. Note that, a region indicated by the symbol P in Fig. 2 is a plasma generation region where plasma is generated. The plasma generation region P is covered with the housing 210.

The plasma generation unit 21 can include a position adjustment mechanism (not illustrated) that adjusts the distance between the aqueous solution and the plasma generation unit 21. The position adjustment mechanism can be configured to be able to move the position of the plasma generation unit 21 in each of the x-axis, y-axis, and z-axis directions. In the present embodiment, the direction of plasma irradiation is the -z-axis direction.

Note that, for the detailed configuration of the plasma generation unit 21, JP 2009-87697 A, JP 2016-150923 A, and the like can be referred to, and the techniques of each of these publications can be incorporated into the present disclosure as necessary.

Further, Fig. 1 shows an example in which the aqueous solution storage tank 10 is configured to store the aqueous solution serving as a raw material before plasma irradiation. The aqueous solution storage tank 10 may include an aqueous solution preparation unit that prepares an aqueous solution by mixing a first aqueous solution that contains sodium lactate or the like but does not contain a surfactant and a second aqueous solution that contains a surfactant but does not contain sodium lactate or the like, or prepares an aqueous solution by adding a surfactant to the first aqueous solution, and may be configured to store the aqueous solution prepared in the aqueous solution preparation unit.

Since the plasma-irradiated aqueous solution generation device 1 of the present embodiment has the above configuration, it is possible to generate the plasma-irradiated aqueous solution of the present embodiment that exhibits a high sterilization effect against true fungi or fungi.

Other configurations and operational effects are the same as those described in the description of the plasma-irradiated aqueous solution of Embodiment 1, and it is also possible to appropriately refer to the description of other embodiments.

### (Embodiment 4)

A sterilization method of Embodiment 4 will be described. The sterilization method of the present embodiment is a method of bringing a plasma-irradiated aqueous solution into contact with true fungi or fungi to sterilize the true fungi or fungi. In the sterilization method of the present embodiment, as the plasma-irradiated aqueous solution, the plasma-irradiated aqueous solution of the present embodiment, the plasma-irradiated aqueous solution obtained by the method for producing a plasma-irradiated aqueous solution of the present embodiment, or the plasma-irradiated aqueous solution generated by the plasma-irradiated aqueous solution generation device of the present embodiment is used. In other words, in the sterilization method of the present embodiment, these plasma-irradiated aqueous solutions are used as an aqueous solution for sterilization.

The method for bringing the plasma-irradiated aqueous solution into contact with true fungi or fungi is not particularly limited, and any method may be used as long as the plasma-irradiated aqueous solution can be brought into contact with a place to be sterilized where true fungi or fungi exist. Examples of the method of bringing the plasma-irradiated aqueous solution into contact with true fungi or fungi include a method of spraying the plasma-irradiated aqueous solution onto true fungi or fungi, a method of applying the plasma-irradiated aqueous solution to true fungi or fungi, a method of applying an impregnated material (for example, gauze, absorbent cotton, and the like) impregnated with the plasma-irradiated aqueous solution to true fungi or fungi, and a method of impregnating an adhered material to which true fungi or fungi adhere into the plasma-irradiated aqueous solution.

The time for which the plasma-irradiated aqueous solution is brought into contact with true fungi or fungi can be preferably 1 hour or more, more preferably 3 hours or more, and still more preferably 6 hours or more, from the viewpoint of enhancing the sterilization effect against true fungi or fungi. The time for which the plasma-irradiated aqueous solution is brought into contact with true fungi or fungi can be preferably 48 hours or less, more preferably 36 hours or less, and still more preferably 24 hours or less, from the viewpoint of the influence on a living body (host) to be treated, such as a plant or a human body.

In the sterilization method of the present embodiment, the plasma-irradiated aqueous solution of the present embodiment, the plasma-irradiated aqueous solution obtained by the method for producing a plasma-irradiated aqueous solution of the present embodiment, or the plasma-irradiated aqueous solution generated by the plasma-irradiated aqueous solution generation device of the present embodiment is brought into contact with true fungi or fungi. Therefore, the sterilization method of the present embodiment can efficiently sterilize true fungi or fungi.

Other configurations and operational effects are the same as those described in the description of the plasma-irradiated aqueous solution of Embodiment 1, and it is also possible to appropriately refer to the description of other embodiments.

Hereinafter, the plasma-irradiated aqueous solution, the method for producing the same, the plasma-irradiated aqueous solution generation device, and the sterilization method of the present embodiment will be described in more detail using experimental examples.

### (Experimental Example 1) Experiment on sterilization of fungal (Penicillium digitatum) spores by plasma-irradiated aqueous solution obtained by irradiating sodium lactate Ringer's solution with plasma

### 1.1 Preparation of Penicillium digitatum spore suspension

Spores were extracted from Penicillium digitatum grown on the potato dextrose agar medium (PDA agar medium). Next, 10 mg of the extracted Penicillium digitatum spores were weighed into a 1.5 mL tube. Next, 1 mL of an aqueous solution obtained by adding 1% by volume of polyoxyethylene sorbitan (20) Monolaurate (Tween20) to distilled water (hereinafter, abbreviated as "1% Tw20") was added thereto to prepare a spore suspension (10 mg/mL). Next, a new 1.5 mL tube containing 900 µL of 1% Tw20 was prepared, and 100 µL of the spore suspension was added thereto to prepare a 10-fold diluted solution. Next, a 10²-fold diluted solution and a 10³-fold diluted solution were prepared in the same manner as described above.

### 1.2 Preparation of plasma-irradiated aqueous solution

A sodium lactate Ringer's solution ("Lactec (registered trademark)" manufactured by Otsuka Pharmaceutical Factory, Inc.) (hereinafter, abbreviated as "Lac") was prepared. The prepared Lac was irradiated with non-equilibrium atmospheric pressure plasma by Ar gas using the above-described plasma irradiation device. A plasma irradiation width was set to 0.3 mm × 20 mm. A flow rate of Ar gas was set to 2 slm. The applied voltage was set to 9 kV, and the frequency was set to 60 Hz. The volume of the solution was set to 8 mL. The plasma irradiation distance, which is the distance from a plasma irradiation port to a liquid surface, was set to 3 mm. The plasma irradiation time was set to 5 minutes irradiation ×2 times. Thus, a conventional plasma-irradiated aqueous solution (hereinafter, abbreviated as "2 × P-Lac") was prepared by irradiating the sodium lactate Ringer solution with plasma. Then, an aqueous solution obtained by mixing Lac and 1% Tw20 (hereinafter, abbreviated as "Lac + 1% Tw20") was prepared. Further, an aqueous solution obtained by mixing 2 × P-Lac and 1% Tw20 (hereinafter, abbreviated as "2 × P-Lac + 1% Tw20") was prepared.

### 1.3 Solution treatment and calculation of viable cell count

A 14 mL culture tube was prepared, and 150 µL of a spore suspension diluted 10³ times was added to 2.85 mL of each of the following three solutions to make a total of 3 mL. These culture tubes were shaken and cultured at room temperature for up to 24 hours.

### (Two types of solutions used in Experimental Example 1)

- Lac + 1% Tw20
- 2 × P-Lac + 1% Tw20

A new 1.5 mL tube was prepared, and 200 µL of the spore suspension shaken and cultured as described above was added to 0.8 mL of each of the three solutions above to make a total of 1 mL.

200 µL each from the culture tube and 1.5 mL tube was applied to the PDA agar medium. Then, the PDA agar medium was allowed to stand on a culture shelf at 25°C. This operation was performed at the start of treatment (0 hours), after 6 hours, and after 24 hours.

The above was cultured for 3 days, and the number of colonies formed was measured (one that that was adjusted to be in the quantitative range of 30 to 300 was adopted). The obtained numerical value was converted to be the viable cell count per 1 mL of solution in each culture tube. As described above, solution treatments for Penicillium digitatum spores with the above various solutions and the calculation of the viable cell count were performed.

### 1.4 Test results and discussion

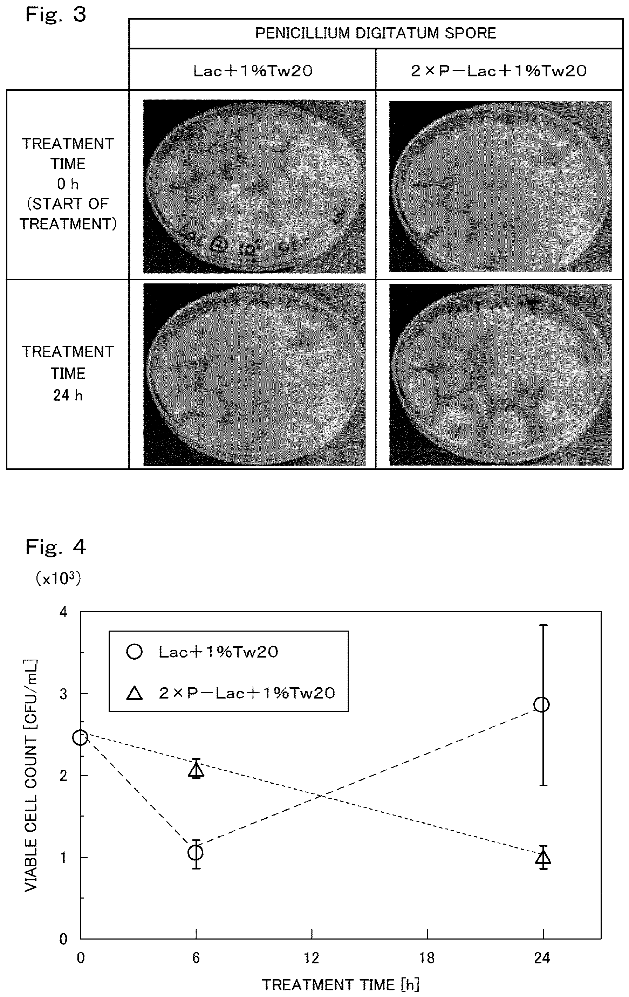

Figs. 3 and 4 show the results of a sterilization test for Penicillium digitatum spores in this experimental example.

It is known that direct plasma irradiation can sterilize Penicillium digitatum spores because the internal structure of cells is oxidatively decomposed by active oxygen species emitted from plasma. It is also known that a conventional plasma-irradiated aqueous solution (2 × P-Lac) obtained by irradiating the sodium lactate Ringer's solution (Lac) with plasma can selectively kill cancer cells.

However, as shown in Figs. 3 and 4, even when Penicillium digitatum spores were treated with a conventional plasma-irradiated aqueous solution (2 × P-Lac) that has a killing effect on cancer cells and to which 1% Tw20 was additionally added, no significant sterilization effect was observed. From this result, it was confirmed that even when a conventional plasma-irradiated aqueous solution obtained by irradiating an aqueous solution that contains sodium lactate but does not contain a surfactant with plasma, or a solution obtained by adding a surfactant after plasma irradiation was used, a high sterilization effect against true fungi or fungi was not achieved (the viable cell count did not decrease by even one digit even after shaking for 24 hours).

### (Experimental Example 2) Experiment on sterilization of fungal (Penicillium digitatum) spores by plasma-irradiated aqueous solution obtained by irradiating aqueous solution containing sodium lactate and surfactant with plasma

### 2.1 Preparation of Penicillium digitatum spore suspension

A spore suspension diluted 10³ times was prepared in the same manner as in Experimental Example 1.

### 2.2 Preparation of plasma-irradiated aqueous solution

An aqueous solution containing sodium lactate and a surfactant (hereinafter, abbreviated as "Na lactate + 1% Tw20") was prepared by mixing 27.6 mM sodium lactate and 1% Tw20. The prepared Na lactate + 1% Tw20 was irradiated with non-equilibrium atmospheric pressure plasma by Ar gas using the above-described plasma irradiation device. A plasma irradiation width was set to 0.3 mm × 20 mm. A flow rate of Ar gas was set to 2 slm. The applied voltage was set to 9 kV, and the frequency was set to 60 Hz. The volume of the solution was set to 8 mL. The plasma irradiation distance, which is the distance from a plasma irradiation port to a liquid surface, was set to 3 mm. The plasma irradiation time was set to 5 minutes irradiation ×2 times. Thus, a plasma-irradiated aqueous solution (hereinafter, abbreviated as "P- (Na lactate + 1% Tw20)") was prepared by irradiating Na lactate + 1% Tw20 with plasma.

Further, a plasma-irradiated aqueous solution (hereinafter, abbreviated as "P-1% Tw20") was prepared by irradiating 1% Tw20 with plasma in the same manner, except that 1% Tw20 was used instead of Na lactate + 1% Tw20.

### 2.3 Solution treatment and calculation of viable cell count

A 14 mL culture tube was prepared, and 150 µL of a spore suspension diluted 10³ times was added to 2.85 mL of each of the following two solutions to make a total of 3 mL. These culture tubes were left to stand at room temperature for up to 24 hours.

### (Four types of solutions used in Experimental Example 2)

- Na lactate + 1% Tw20
- P- (Na lactate + 1% Tw20)
- 1% Tw20
- P-1% Tw20

250 µL was taken out from this culture tube, and 200 µL was applied to the PDA agar medium. The remaining 50 µL was diluted 10 times with 450 µL of 1% Tw20 solution, and 200 µL of that was applied to the PDA agar medium. Then, the PDA agar medium was allowed to stand on a culture shelf at 25°C. This operation was performed at the start of treatment (0 hours), after 1 hour, after 5 hours, and after 24 hours.

The above was cultured for 3 days, and the number of colonies formed was measured (one that was adjusted to be in the quantitative range of 30 to 300 was adopted). The obtained numerical value was converted to be the viable cell count per 1 mL of solution in each culture tube. As described above, solution treatments for Penicillium digitatum spores with the above various solutions and the calculation of the viable cell count were performed.

### 2.4 Test results and discussion

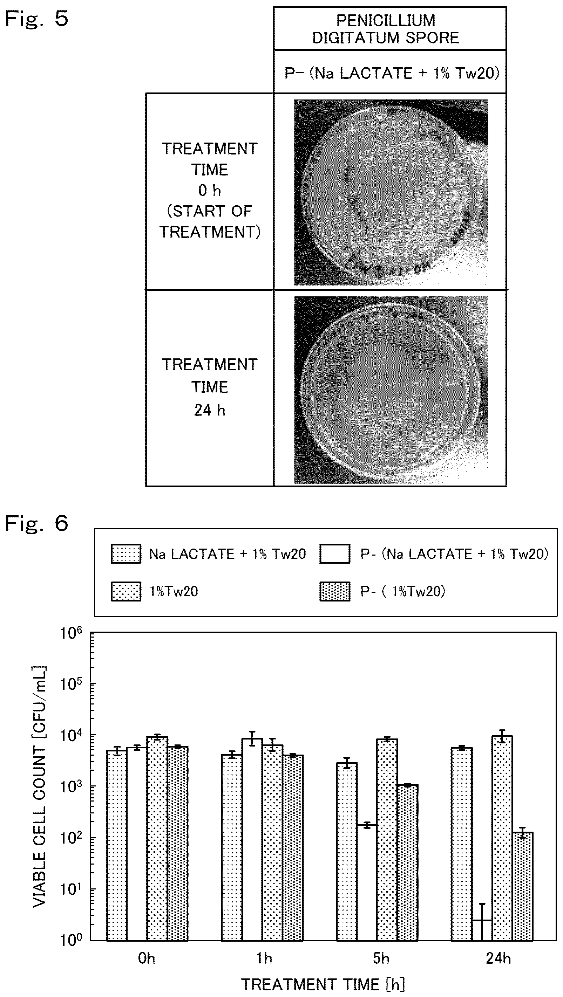

Figs. 5 and 6 show the results of the sterilization test for Penicillium digitatum spores in this experimental example. Note that, in Fig. 6, upper and lower values of n=2 are used as error bars.

As shown in Figs. 5 and 6, by treating Penicillium digitatum spores with a plasma-irradiated aqueous solution (P- (Na lactate + 1% Tw20)) obtained by irradiating an aqueous solution containing sodium lactate and a surfactant with plasma, a significant sterilization effect of two orders of magnitude after 5 hours and three orders of magnitude after 24 hours was observed. Note that, also in the case where Penicillium digitatum spores were treated with a plasma-irradiated aqueous solution (P-1% Tw20) obtained by irradiating an aqueous solution containing only a surfactant without sodium lactate with plasma, a sterilization effect was observed, but it was found that the effect was one order of magnitude weaker than that of a plasma-irradiated aqueous solution (P- (Na lactate + 1% Tw20)) obtained by irradiating an aqueous solution containing sodium lactate and a surfactant with plasma.

Note that, as a result of Digital Pack Test ¹, P- (Na lactate + 1% Tw20) contained 1858.8 µM of H₂O₂. Further, the pH of 6.45 before plasma irradiation was changed to pH 4.64 after plasma irradiation. Similarly, P-1% Tw20 contained 964.7 µM of H₂O₂. Further, the pH of 6.00 before plasma irradiation was changed to pH 2.80 after plasma irradiation.

### (Experimental Example 3) Investigation of concentration dependence of surfactant in plasma-irradiated aqueous solution

### 3.1 Preparation of Penicillium digitatum spore suspension

A spore suspension diluted 10³ times was prepared in the same manner as in Experimental Example 1.

### 3.2 Preparation of surfactant-containing aqueous solution

The following surfactant-containing aqueous solutions with different concentrations of polyoxyethylene (20) Sorbitan Monolaurate (Tween20) were prepared.
- An aqueous solution (1% Tw20) obtained by adding 1% by volume of polyoxyethylene (20) Sorbitan Monolaurate (Tween20) to distilled water
- An aqueous solution obtained by adding 0.1% by volume of polyoxyethylene (20) Sorbitan Monolaurate (Tween20) to distilled water (hereinafter, abbreviated as "0.1% Tw20")
- An aqueous solution obtained by adding 0.01% by volume of polyoxyethylene (20) Sorbitan Monolaurate (Tween20) to distilled water (hereinafter, abbreviated as "0.01% Tw20")

### 3.3 Preparation of plasma-irradiated aqueous solution

According to Experimental Example 2, sodium lactate and 1% Tw20 were mixed to prepare an aqueous solution containing sodium lactate and 1% by volume of a surfactant (Na lactate + 1% Tw20). By irradiating this with non-equilibrium atmospheric pressure plasma, a plasma-irradiated aqueous solution by irradiating Na lactate + 1% Tw20 with plasma (P- (Na lactate + 1% Tw20)) was prepared.

In the same manner as described above, sodium lactate and 0.1% Tw20 were mixed to prepare an aqueous solution containing sodium lactate and 0.1% by volume of a surfactant (hereinafter, abbreviated as "Na lactate + 0.1% Tw20"). By irradiating this with non-equilibrium atmospheric pressure plasma, a plasma-irradiated aqueous solution by irradiating Na lactate + 0.1% Tw20 with plasma (P- (Na lactate + 0.1% Tw20)) was prepared.

Further, sodium lactate and 0.01% Tw20 were mixed to prepare an aqueous solution containing sodium lactate and 0.01% by volume of a surfactant (hereinafter, abbreviated as "Na lactate + 0.01% Tw20"). By irradiating this with non-equilibrium atmospheric pressure plasma, a plasma-irradiated aqueous solution by irradiating Na lactate + 0.01% Tw20 with plasma (P- (Na lactate + 0.01% Tw20)) was prepared.

### 3.4 Solution treatment and calculation of viable cell count

According to Experimental Example 2, solution treatments for Penicillium digitatum spores with the following four types of solutions were performed, and the viable cell count was calculated. Note that, to each of the solutions of P- (Na lactate + 0.1% Tw20) and P- (Na lactate + 0.01% Tw20), untreated Tween20 was added so as to be a total of 1% by volume after plasma irradiation, and then treatment with Penicillium digitatum spores was performed.

### (Four types of solutions used in Experimental Example 3)

- Na lactate + 1% Tw20
- P- (Na lactate + 1% Tw20)
- P- (Na lactate + 0.1% Tw20)
- P- (Na lactate + 0.01% Tw20)

### 3.5 Test results and discussion

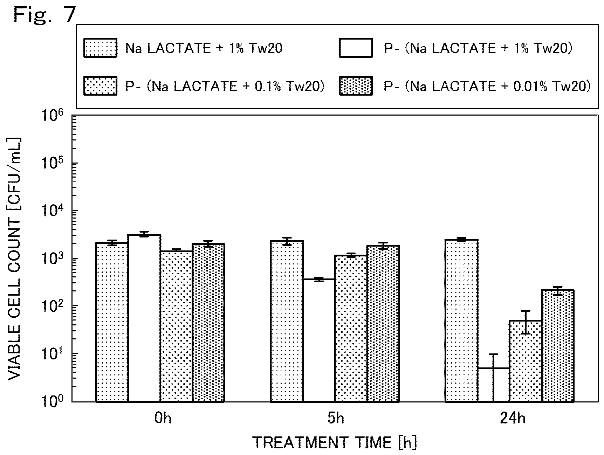

Fig. 7 shows the results of a test on the concentration dependence of the surfactant in the plasma-irradiated aqueous solution. Note that, in Fig. 7, the upper and lower values of n=2 are used as error bars.

As shown in Fig. 7, it was confirmed that the greater the concentration of the surfactant in the plasma-irradiated aqueous solution, the higher the sterilization effect against true fungi or fungi. Further, it was found that a sufficient sterilization effect can be achieved when the concentration of the surfactant in the plasma-irradiated aqueous solution is 1% by volume or less.

### (Experimental Example 4) Experiment on sterilization of fungal (Penicillium digitatum) spores by plasma-irradiated aqueous solution using various surfactants

Each experimental example described above was a sterilization experiment using polyoxyethylene (20) Sorbitan Monolaurate (Tween20), being a nonionic surfactant, as a surfactant to be contained in the aqueous solution before plasma irradiation. In this experimental example, a sterilization experiment was performed using other various surfactants than Tween20.

### 4.1 Preparation of Penicillium digitatum spore suspension

A spore suspension diluted 10³ times was prepared in the same manner as in Experimental Example 1.

### 4.2 Preparation of surfactant-containing aqueous solution

The following surfactant-containing aqueous solutions containing different types of surfactants were prepared. Note that, the concentration of each surfactant was selected with reference to the concentration range actually used in the medical settings.
- An aqueous solution (1% Tw20) obtained by adding 1% by volume of polyoxyethylene (20) Sorbitan Monolaurate (Tween20) as a nonionic surfactant to distilled water
- An aqueous solution obtained by adding 0.01% by volume of benzalkonium chloride as a cationic surfactant to distilled water (hereinafter, abbreviated as "0.01% benzalkonium chloride")
- An aqueous solution obtained by adding 1% by volume of sodium dodecyl sulfate (SDS) as an anionic surfactant to distilled water (hereinafter, abbreviated as "1% SDS")
- An aqueous solution obtained by adding 0.02% by volume of alkylpolyamino ethylglycine hydrochloride as an amphoteric surfactant to distilled water (hereinafter, abbreviated as "0.02% alkylpolyamino ethylglycine hydrochloride").

### 4.3 Preparation of plasma-irradiated aqueous solution

According to Experimental Example 2, sodium lactate and 1% Tw20 were mixed to prepare an aqueous solution containing sodium lactate and 1% by volume of a surfactant (Na lactate + 1% Tw20). By irradiating this with non-equilibrium atmospheric pressure plasma, a plasma-irradiated aqueous solution by irradiating Na lactate + 1% Tw20 with plasma (P- (Na lactate + 1% Tw20)) was prepared.

In the same manner as described above, sodium lactate and 0.01% benzalkonium chloride were mixed to prepare an aqueous solution containing sodium lactate and 0.01% by volume of benzalkonium chloride (hereinafter, abbreviated as "Na lactate + 0.01% benzalkonium chloride"). By irradiating this with non-equilibrium atmospheric pressure plasma, a plasma-irradiated aqueous solution by irradiating Na lactate + 0.01% benzalkonium chloride with plasma (P- (Na lactate + 0.01% benzalkonium chloride)) was prepared.

Further, sodium lactate and 1% SDS were mixed to prepare an aqueous solution containing sodium lactate and 1% by volume of SDS (hereinafter, abbreviated as "Na lactate + 1% SDS"). By irradiating this with non-equilibrium atmospheric pressure plasma, a plasma-irradiated aqueous solution by irradiating Na lactate + 1% SDS with plasma (P- (Na lactate + 1% SDS)) was prepared.

Further, sodium lactate and 0.02% alkylpolyamino ethylglycine hydrochloride were mixed to prepare an aqueous solution containing sodium lactate and 0.02% by volume of alkylpolyamino ethylglycine hydrochloride (hereinafter, abbreviated as "Na lactate + 0.02% alkylpolyamino ethylglycine hydrochloride"). By irradiating this with non-equilibrium atmospheric pressure plasma, a plasma-irradiated aqueous solution by irradiating Na lactate + 0.02% alkylpolyamino ethylglycine hydrochloride with plasma (P-(Na lactate + 0.02% alkylpolyamino ethylglycine hydrochloride)) was prepared.

### 4.4 Solution treatment and calculation of viable cell count

According to Experimental Example 2, solution treatments of Penicillium digitatum spores with the following eight types of solutions were performed, and the viable cell count was calculated.

### (Eight types of solutions used in Experimental Example 4)

- Na lactate + 1% Tw20
- P- (Na lactate + 1% Tw20)
- Na lactate + 0.01% benzalkonium chloride
- P- (Na lactate + 0.01% benzalkonium chloride)
- Na lactate + 1% SDS
- P- (Na lactate + 1% SDS)
- Na lactate + 0.02% alkylpolyamino ethylglycine hydrochloride
- P- (Na lactate + 0.02% alkylpolyamino ethylglycine hydrochloride)

### 4.5 Test results and discussion

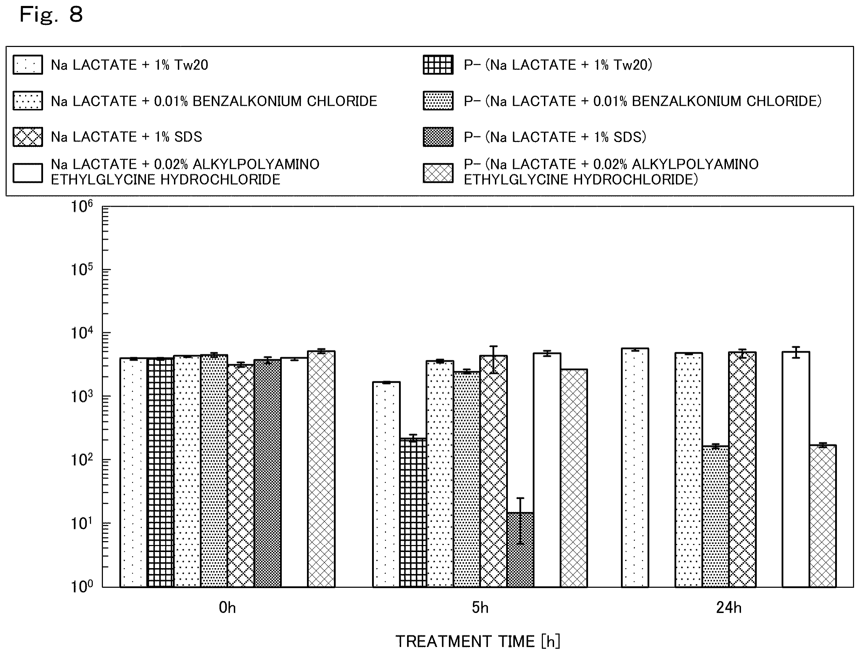

Fig. 8 shows the results of sterilization experiments of fungal (Penicillium digitatum) spores by plasma-irradiated aqueous solutions using various surfactants. Note that, in Fig. 8, the upper and lower values of n=2 are used as error bars.

As shown in Fig. 8, it was confirmed that not only a nonionic surfactant (Tween20 in this experimental example) but also a cationic surfactant (benzalkonium chloride in this experimental example), an anionic surfactant (sodium dodecyl sulfate in this experimental example), and an amphoteric surfactant (alkylpolyamino ethylglycine hydrochloride in this experimental example) all exhibit a sterilization effect against true fungi or fungi when mixed with sodium lactate and subjected to plasma irradiation.

Further, the concentrations used in the medical settings of a cationic surfactant (benzalkonium chloride) or an amphoteric surfactant (alkylpolyamino ethylglycine hydrochloride), which themselves have a sterilization effect, are lower than those of a nonionic surfactant ((Tween20) in consideration of damage and injury to the human body, such as the skin, but it was confirmed that they exhibit a sterilization effect against true fungi or fungi even at such low concentrations.

### (Experimental Example 5) Experiment on sterilization against other true fungi other than Penicillium digitatum spores - Aspergillus flavus spores

### 5.1 Preparation of Aspergillus flavus (A. flavus) spore suspension

An Aspergillus flavus (A. flavus) spore suspension was prepared in the same manner as in Experimental Example 2.

### 5.2 Preparation of plasma-irradiated aqueous solution

P- (Na lactate + 1% Tw20) was prepared in the same manner as in Experimental Example 2.

### 5.3 Solution treatment and calculation of viable cell count

In the same manner as in Experimental Example 2, the Aspergillus flavus (A. flavus) spore suspension was mixed with the following various solutions, treated for 24 hours, and the viable cell count was calculated.

### (Two types of solutions used in Experimental Example 5)

- Na lactate + 1% Tw20
- P- (Na lactate + 1% Tw20)

### 5.4 Test results and discussion

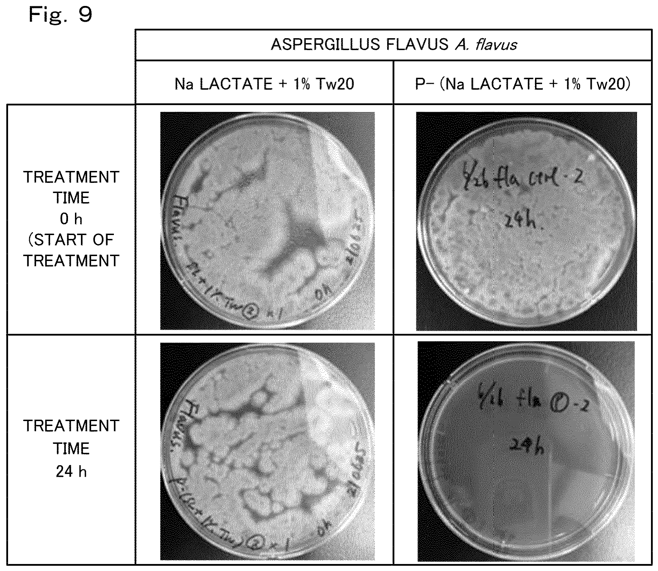

Fig. 9 shows the results of the sterilization test for Aspergillus flavus (A. flavus) spores in this experimental example. As shown in Fig. 9, it was confirmed that the plasma-irradiated aqueous solution according to the present disclosure exhibits a sterilization effect against not only Penicillium digitatum spores but also Aspergillus flavus (A. flavus) spores that produce carcinogenic toxin aflatoxin.

### (Experimental Example 6) Experiment on sterilization against other true fungi other than Penicillium digitatum spores - Aspergillus niger spores -

### 6.1 Preparation of Aspergillus niger (A. niger) spore suspension

An Aspergillus niger (A. niger) spore suspension was prepared in the same manner as in Experimental Example 2.

### 6.2 Preparation of plasma-irradiated aqueous solution

P- (Na lactate + 1% Tw20) was prepared in the same manner as in Experimental Example 2. Also, P- (Na lactate + 1% SDS) was prepared in the same manner as in Experimental Example 4.

### 6.3 Solution treatment and calculation of viable cell count

In the same manner as in Experimental Example 2, the Aspergillus niger (A. niger) spore suspension was mixed with the following various solutions, treated for 24 hours, and the viable cell count was calculated.

### (Four types of solutions used in Experimental Example 6)

- Na lactate + 1% Tw20
- P- (Na lactate + 1% Tw20)
- Na lactate + 1% SDS
- P- (Na lactate + 1% SDS)

### 6.4 Test results and discussion

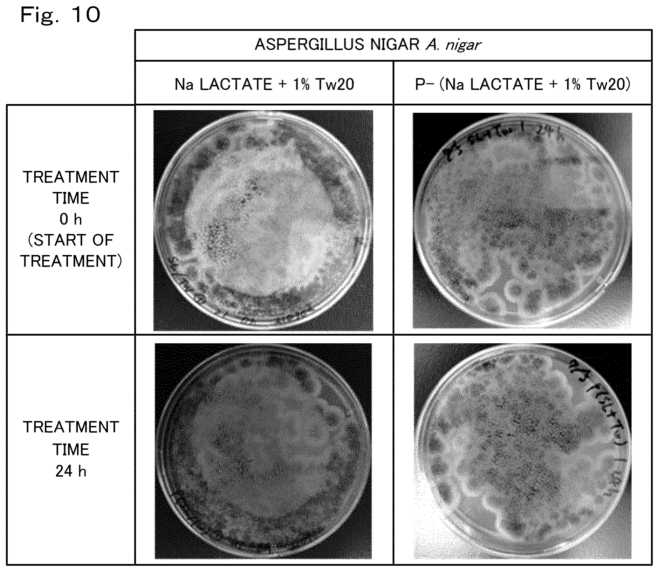

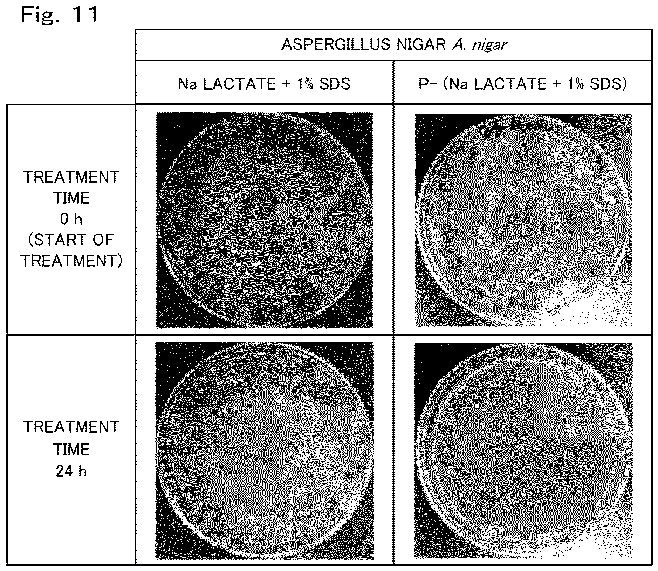

Figs. 10 and 11 show the results of the sterilization test for Aspergillus niger (A. niger) spores in this experimental example. As shown in Figs. 10 and 11, it was confirmed that the plasma-irradiated aqueous solution according to the present disclosure exhibits a sterilization effect also against Aspergillus niger (A. niger) spores that produce mycotoxins. Further, as shown in Figs. 10 and 11, it was suggested that SDS has a larger effect contributing to sterilization than Tween20.

### (Experimental Example 7) Experiment on sterilization against other true fungi other than Penicillium digitatum spores - Glomerella acutata spores

### 7.1 Preparation of Glomerella acutata spore suspension

A Glomerella acutata spore suspension was prepared in the same manner as in Experimental Example 2.

### 7.2 Preparation of plasma-irradiated aqueous solution

P- (Na lactate + 1% Tw20) was prepared in the same manner as in Experimental Example 2.

### 7.3 Solution treatment and calculation of viable cell count

In the same manner as in Experimental Example 2, the Glomerella acutata spore suspension was mixed with the following various solutions, treated for 3 hours, and the viable cell count was calculated.

### (Three types of solutions used in Experimental Example 7)

- Na lactate + 1% Tw20
- P- (Na lactate + 1% Tw20)
- Sterile water (control group)

### 7.4 Test results and discussion

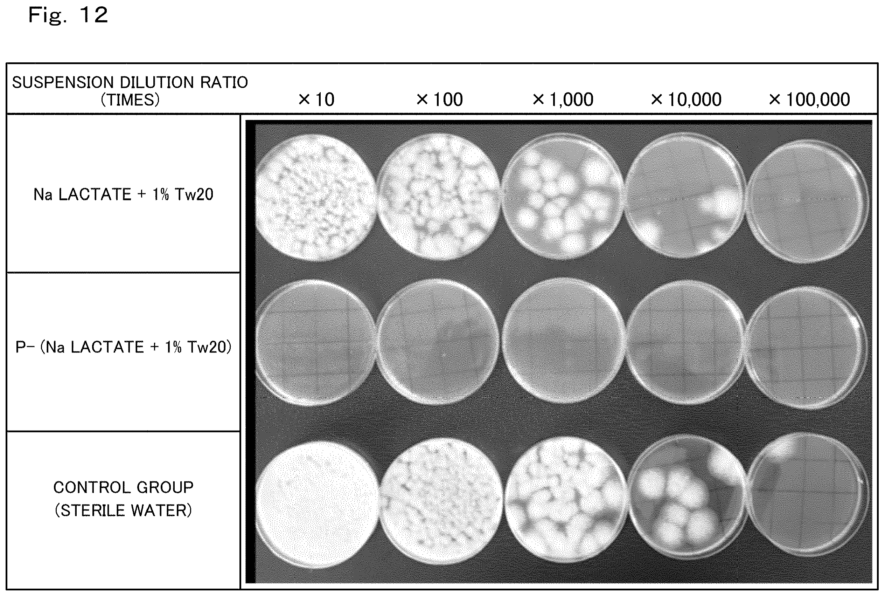

Fig. 12 shows the results of the sterilization test for Glomerella acutata spore in this experimental example. As shown in Fig. 12, it was confirmed that the plasma-irradiated aqueous solution according to the present disclosure exhibits a strong sterilization effect also against Glomerella acutata spores that cause severe damage to crop cultivation such as strawberries.

### (Experimental Example 8) Experiment on sterilization against other true fungi other than Penicillium digitatum spores - Trichophyton spores -

### 8.1 Preparation of Trichophyton spore suspension

3 mL of 1% Tw20 was added to Trichophyton in which a sufficient amount of fungus bodies (>10⁸) were cultured on a PDA agar medium prepared in a φ 9 cm petri dish, and the mixture was stirred well to collect a fungus liquid, and 2 mL of 1% Tw20 was further added. Then, it was filtered with absorbent cotton to collect Trichophyton spores. Then, it was centrifuged (1500×g, 5 min) and the pellet was suspended with 1 mL of 1% Tw20. Thus, a Trichophyton spore suspension was prepared.

### 8.2 Preparation of plasma-irradiated aqueous solution

P- (Na lactate + 1% Tw20) was prepared in the same manner as in Experimental Example 2. Note that, as a result of Digital Pack Test ², in this experimental example, P-(Na lactate + 1% Tw20) contained 1576.5 µM of H₂O₂ and 2087.0 µM of NO₂⁻.

### 8.3 Solution treatment and calculation of viable cell count

A 14 mL culture tube was prepared, and 150 µL of the above-described Trichophyton spore suspension was added to 2.85 mL of each of the following two solutions to make a total of 3 mL. These culture tubes were shaken and cultured at room temperature for up to 24 hours.

### (Two types of solutions used in Experimental Example 8)

- Na lactate + 1% Tw20
- P- (Na lactate + 1% Tw20)

Samples were taken from those with a treatment time of 0 hours and a treatment time of 24 hours, a dilution stage was made up to 10⁶ times from the stock solution, and 100 µL of each was applied to a BD-Rodac plate. Note that, in the case of the treatment time of 0 hours, only those that were not irradiated with plasma were performed.

The above was cultured for 5 days, and the number of colonies formed was measured and the viable cell count was calculated in the same manner as in Experimental Example 2.

### 8.4 Test results and discussion

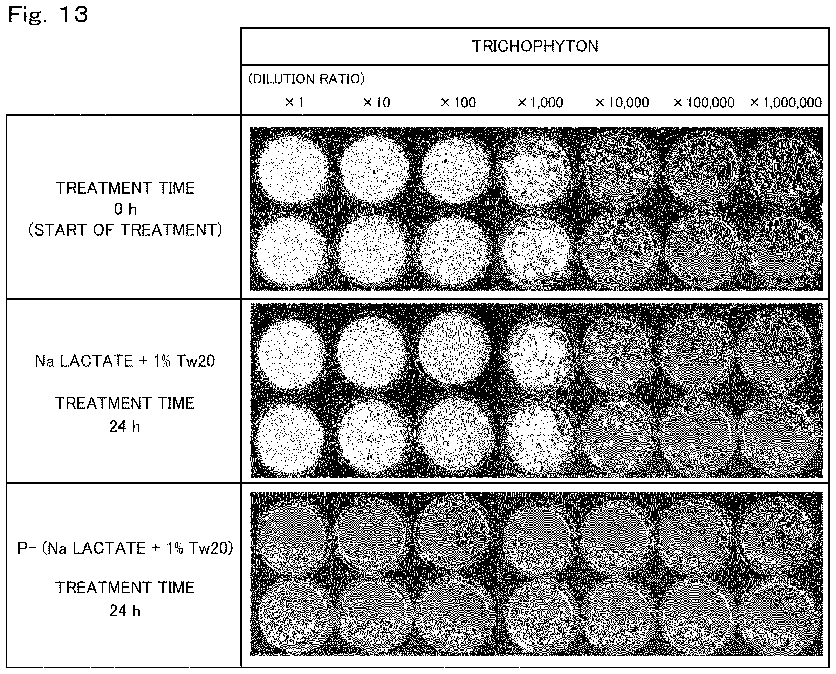

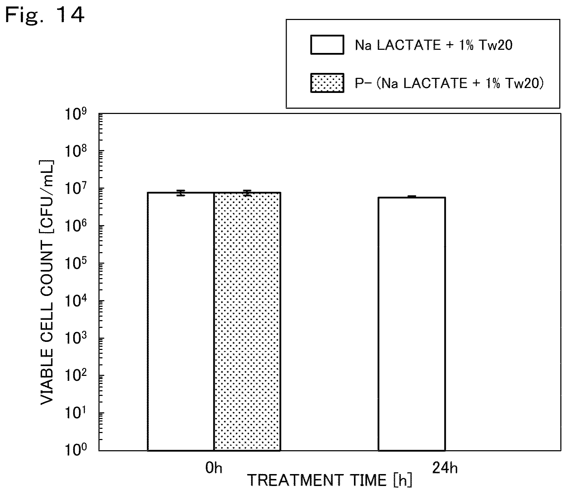

Figs. 13 and 14 show the results of the sterilization test for Trichophyton in this experimental example. Note that, in Fig. 14, the upper and lower values of n=2 are used as error bars.

As shown in Figs. 13 and 14, it was confirmed that the plasma-irradiated aqueous solution according to the present disclosure exhibited a strong sterilization effect against Trichophyton, and six to seven digit numbers of the number of fungi was completely sterilized.

### (Experimental Example 9) Experiment on sterilization of fungal (Penicillium digitatum) spores by plasma-irradiated aqueous solution using surfactant used for pharmaceuticals

### 9.1 Preparation of Penicillium digitatum spore suspension

Spore suspensions diluted 10² times and 10³ times were prepared in the same manner as in Experimental Example 1.

### 9.2 Preparation of plasma-irradiated aqueous solution

The following surfactant-containing aqueous solutions containing various surfactants used for pharmaceuticals were prepared. Note that, the concentration of each surfactant was selected with reference to the concentration range actually used in the medical settings.
- An aqueous solution (1% Tw20) obtained by adding 1% by volume of polyoxyethylene (20) Sorbitan Monolaurate (Tween20) to distilled water
- An aqueous solution obtained by adding 1% by volume of polyoxyethylene (20) Sorbitan Monooleate (Tween80) to distilled water (hereinafter, abbreviated as "1% Tw80")
- An aqueous solution obtained by adding 0.1% by volume of sodium alkylbenzene sulfonate (LAS) to distilled water (hereinafter, abbreviated as "0.1% LAS")
- An aqueous solution obtained by adding 1% by volume of sodium alkylbenzene sulfonate (LAS) to distilled water (hereinafter, abbreviated as "1% LAS")
- An aqueous solution obtained by adding 0.1% by volume of polyoxyethylene hydrogenated castor oil 60 to distilled water (hereinafter, abbreviated as "0.1% castor oil")
- An aqueous solution obtained by adding 1% by volume of polyoxyethylene hydrogenated castor oil 60 to distilled water (hereinafter, abbreviated as "1% castor oil")

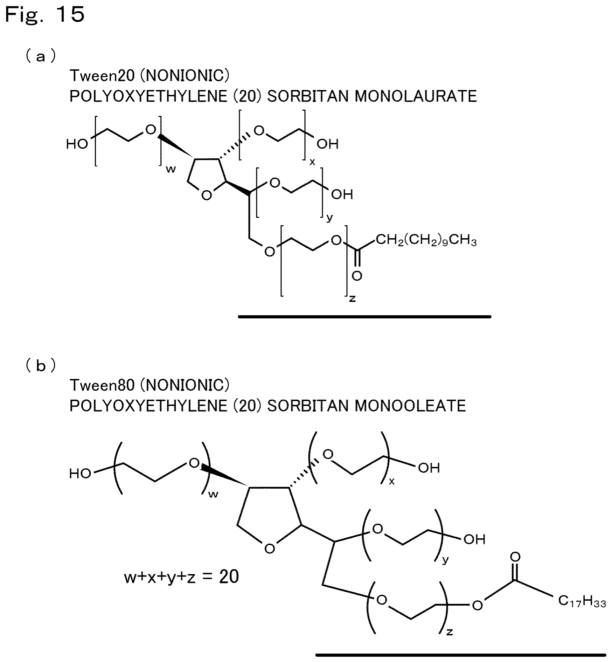

Note that, polyoxyethylene (20) Sorbitan Monolaurate (Tween20) and polyoxyethylene (20) Sorbitan Monooleate (Tween80) have different molecular structures in the underlined part as shown in Fig. 15.

### 9.3 Preparation of plasma-irradiated aqueous solution

According to Experimental Example 2, sodium lactate and 1% Tw20 were mixed to prepare an aqueous solution containing sodium lactate and 1% by volume of Tween20 (Na lactate + 1% Tw20). By irradiating this with non-equilibrium atmospheric pressure plasma, a plasma-irradiated aqueous solution by irradiating Na lactate + 1% Tw20 with plasma (P- (Na lactate + 1% Tw20)) was prepared.

In the same manner as described above, sodium lactate and 1% Tw80 were mixed to prepare an aqueous solution containing sodium lactate and 1% by volume of Tween80 (Na lactate + 1% Tw80). By irradiating this with non-equilibrium atmospheric pressure plasma, a plasma-irradiated aqueous solution by irradiating Na lactate + 1% Tw80 with plasma (P- (Na lactate + 1% Tw80)) was prepared.

Further, sodium lactate and 0.1% LAS were mixed to prepare an aqueous solution containing sodium lactate and 0.1% by volume of LAS (Na lactate + 0.1% LAS). By irradiating this with non-equilibrium atmospheric pressure plasma, a plasma-irradiated aqueous solution by irradiating Na lactate + 0.1% LAS with plasma (P- (Na lactate + 0.1% LAS)) was prepared.

Further, sodium lactate and 1% LAS were mixed to prepare an aqueous solution containing sodium lactate and 1% by volume of LAS (Na lactate + 1% LAS). By irradiating this with non-equilibrium atmospheric pressure plasma, a plasma-irradiated aqueous solution by irradiating Na lactate + 1% LAS with plasma (P- (Na lactate + 1% LAS)) was prepared.

Further, sodium lactate and 0.1% castor oil were mixed to prepare an aqueous solution containing sodium lactate and 0.1% by volume of castor oil (Na lactate + 0.1% castor oil). By irradiating this with non-equilibrium atmospheric pressure plasma, a plasma-irradiated aqueous solution by irradiating Na lactate + 0.1% castor oil with plasma (P- (Na lactate + 0.1% castor oil)) was prepared.

Further, sodium lactate and 1% castor oil were mixed to prepare an aqueous solution containing sodium lactate and 1% by volume of castor oil (Na lactate + 1% castor oil). By irradiating this with non-equilibrium atmospheric pressure plasma, a plasma-irradiated aqueous solution by irradiating Na lactate + 1% castor oil with plasma (P- (Na lactate + 1% castor oil)) was prepared.

### 9.4 Solution treatment and calculation of viable cell count

According to Experimental Example 2, solution treatments for Penicillium digitatum spores with the following nine types of solutions were performed, and the viable cell count was calculated.

### (Nine types of solutions used in Experimental Example 9)

- Na lactate + 1% Tw20
- P- (Na lactate + 1% Tw20)
- P- (Na lactate + 1% Tw80)
- Na lactate + 1% LAS
- P- (Na lactate + 0.1% LAS)
- P- (Na lactate + 1% LAS)
- Na lactate + 1% castor oil
- P- (Na lactate + 0.1% castor oil)
- P- (Na lactate + 1% castor oil)

### 9.5 Test results and discussion

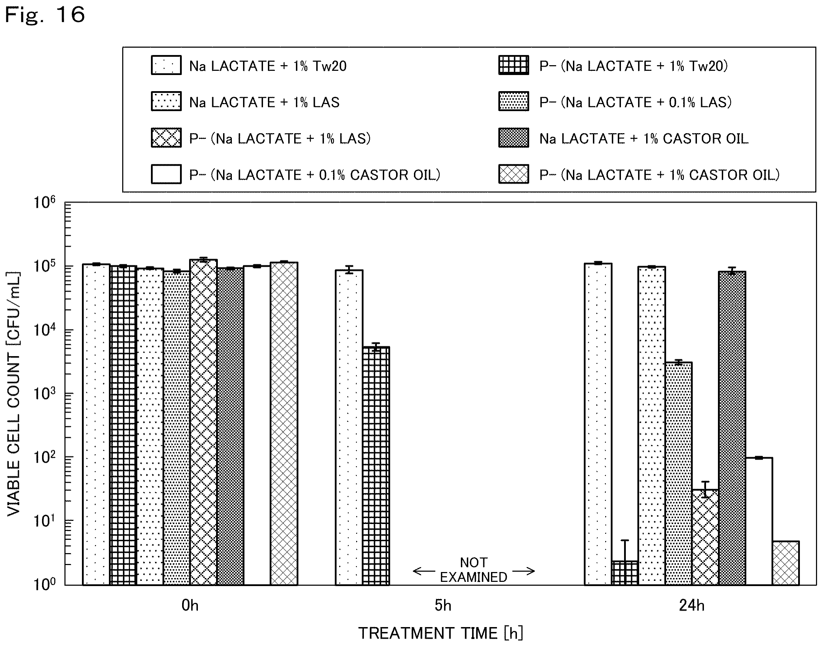

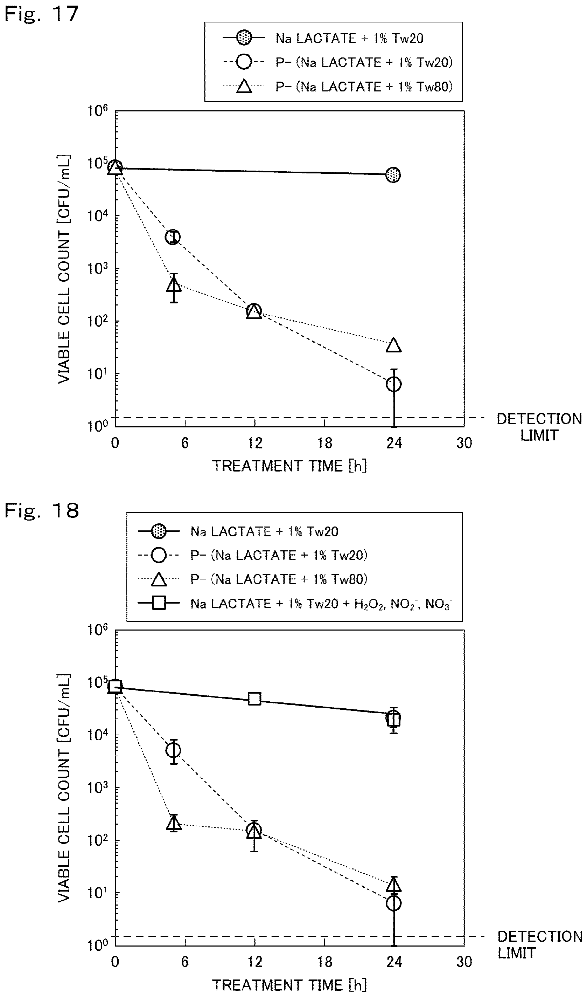

Fig. 16 shows the results of sterilization experiments of fungal (Penicillium digitatum) spores by plasma-irradiated aqueous solutions using various surfactants used for pharmaceuticals. Note that, in Fig. 16, the upper and lower values of n=2 are used as error bars. Further, Fig. 17 shows the results of sterilization experiments of fungal (Penicillium digitatum) spores by a plasma-irradiated aqueous solution using Tween20 as a surfactant and a plasma-irradiated aqueous solution using Tween80 as a surfactant.

As shown in Fig. 16, it was confirmed that both the plasma-irradiated aqueous solution using sodium alkylbenzene sulfonate (LAS) and the plasma-irradiated aqueous solution using polyoxyethylene hydrogenated castor oil exhibited a sterilization effect against true fungi or fungi depending on the concentration of the surfactant contained in the aqueous solution before plasma irradiation.

Further, as shown in Fig. 17, Tween80 has a different molecular structure in the underlined part compared to Tween20 as shown in Fig. 15, but it was confirmed that Tween80 exhibits a sterilization effect against true fungi or fungi, similar to the case of using Tween20.

### (Experimental Example 10) Experiment on confirmation of sterilization effect of reaction products generated by reaction between water and plasma

The plasma-irradiated aqueous solution of the present disclosure is obtained by irradiating an aqueous solution containing at least one selected from the group consisting of sodium lactate, potassium lactate, calcium lactate, and lactic acid, and a surfactant, with plasma. Therefore, the reaction products H₂O₂ (hydrogen peroxide solution), nitrite ions (NO₂⁻), and nitrate ions (NO₃⁻), are generated by the reaction between water contained in the aqueous solution before plasma irradiation and plasma, and these may have a sterilization effect against true fungi or fungi. Therefore, it was decided to confirm the sterilization effect against true fungi or fungi for the aqueous solution to which H₂O₂, NO₂⁻, and NO₃⁻ were added at the same concentration generated by the reaction between water and plasma.

### 10.1 Preparation of Penicillium digitatum spore suspension

A 10²-fold diluted solution thereof was prepared from the spore suspension (10 mg/mL) in the same manner as in Experimental Example 1.

### 10.2 Preparation of plasma-irradiated aqueous solution

According to Experimental Example 2, sodium lactate and 1% Tw20 were mixed to prepare an aqueous solution containing sodium lactate and 1% by volume of Tween20 (Na lactate + 1% Tw20). By irradiating this with non-equilibrium atmospheric pressure plasma, a plasma-irradiated aqueous solution by irradiating Na lactate + 1% Tw20 with plasma (P- (Na lactate + 1% Tw20)) was prepared.

In the same manner as described above, sodium lactate and 1% Tw80 were mixed to prepare an aqueous solution containing sodium lactate and 1% by volume of Tween80 (Na lactate + 1% Tw80). By irradiating this with non-equilibrium atmospheric pressure plasma, a plasma-irradiated aqueous solution by irradiating Na lactate + 1% Tw80 with plasma (P- (Na lactate + 1% Tw80)) was prepared.

### 10.3 Preparation of aqueous solution with H₂O₂, NO₂⁻, and NO₃⁻ added

1317.6 µM of H₂O₂ was added to Na lactate + 1% Tw20 from measurement results of the concentration by a digital pack test, 1605.8 µM of NO₂⁻ and 330.8 µM of NO₃⁻ were added based on the measurement results using HPLC, and an aqueous solution containing reaction products with water (hereinafter, abbreviated as "Na lactate + 1% Tw20 + H₂O₂, NO₂⁻, NO₃⁻") was prepared.

### 10.4 Solution treatment and calculation of viable cell count

According to Experimental Example 2, solution treatments for Penicillium digitatum spores with the following four types of solutions were performed, and the viable cell count was calculated.

### (Four types of solutions used in Experimental Example 10)

- Na lactate + 1% Tw20
- P- (Na lactate + 1% Tw20)
- P- (Na lactate + 1% Tw80)
- Na lactate + 1% Tw20 + H₂O₂, NO₂⁻, NO₃⁻

### 10.5 Test results and discussion

Fig. 18 shows the results of an experiment for confirming the sterilization effect of the reaction products generated by the reaction between water and plasma.

As shown in Fig. 18, it was confirmed that H₂O₂, NO₂⁻, and NO₃⁻, which are reaction products generated by the reaction between water and plasma, did not contribute to sterilization against true fungi or fungi at all. From these results, it was confirmed that in preparation of a plasma-irradiated aqueous solution, when an aqueous solution containing a surfactant in addition to sodium lactate or the like is used, and plasma irradiation is performed on the aqueous solution to produce a plasma-irradiated aqueous solution, a significant sterilization effect against true fungi or fungi can be exhibited.

### (Experimental Example 11) Experiment on sterilization of fungal (Penicillium digitatum) spores by plasma-irradiated aqueous solution obtained by irradiating aqueous solution containing alkali metal/alkaline earth metal lactate or lactic acid and surfactant with plasma

### 11.1 Preparation of Penicillium digitatum spore suspension

A 10²-fold diluted solution thereof was prepared from the spore suspension (10 mg/mL) in the same manner as in Experimental Example 1.

### 11.2 Preparation of plasma-irradiated aqueous solution

In the same manner as in Experimental Example 2, an aqueous solution containing sodium lactate and a surfactant (Na lactate + 1% Tw20) was prepared by mixing 27.6 mM sodium lactate and 1% Tw20. By irradiating this with non-equilibrium atmospheric pressure plasma, a plasma-irradiated aqueous solution by irradiating Na lactate + 1% Tw20 with plasma (P- (Na lactate + 1% Tw20)) was prepared.

Further, an aqueous solution containing potassium lactate and a surfactant (K lactate + 1% Tw20) was prepared by mixing 27.6 mM potassium lactate and 1% Tw20. By irradiating this with non-equilibrium atmospheric pressure plasma, a plasma-irradiated aqueous solution by irradiating K lactate + 1% Tw20 with plasma (P- (K lactate + 1% Tw20)) was prepared.

Further, an aqueous solution containing lactic acid and a surfactant (lactic acid + 1% Tw20) was prepared by mixing 27.6 mM lactic acid and 1% Tw20. By irradiating this with non-equilibrium atmospheric pressure plasma, a plasma-irradiated aqueous solution by irradiating lactic acid + 1% Tw20 with plasma (P- (lactic acid + 1% Tw20)) was prepared.

### 11.3 Solution treatment and calculation of viable cell count

According to Experimental Example 2, solution treatments for Penicillium digitatum spores with the following five types of solutions were performed, and the viable cell count was calculated.

### (Five types of solutions used in Experimental Example 11)

- P- (Na lactate + 1% Tw20)
- K lactate + 1% Tw20
- P- (K lactate + 1% Tw20)
- Lactic Acid + 1% Tw20
- P- (Lactic Acid + 1% Tw20)

Note that, as a result of Digital Pack Test ³, Na lactate + 1% Tw20 contained 1388.2 µM of H₂O₂ and 1565.2 µM of NO₂⁻, while NO₃⁻ could not be measured. Further, the pH of Na lactate + 1% Tw20 was changed from 6.61 before plasma irradiation to pH 4.52 after plasma irradiation. Similarly, K lactate + 1% Tw20 contained 1376.5 µM of H₂O₂ and 1913.0 µM of NO₂⁻, while NO₃⁻ could not be measured. Further, the pH of K lactate + 1% Tw20 was changed from 6.28 before plasma irradiation to pH 4.98 after plasma irradiation. Similarly, lactic Acid + 1% Tw20 contained 823.5 µM of H₂O₂, 4.1 µM of NO₂⁻, and 267.7 µM of NO₃⁻. Further, the pH of lactic acid + 1% Tw20 was changed from 2.57 before plasma irradiation to pH 2.42 after plasma irradiation. Note that, in another assay method performed previously, NO₃⁻ in Na lactate + 1% Tw20 and K lactate + 1% Tw20 was 330.8 µM.

### 11.4 Test results and discussion

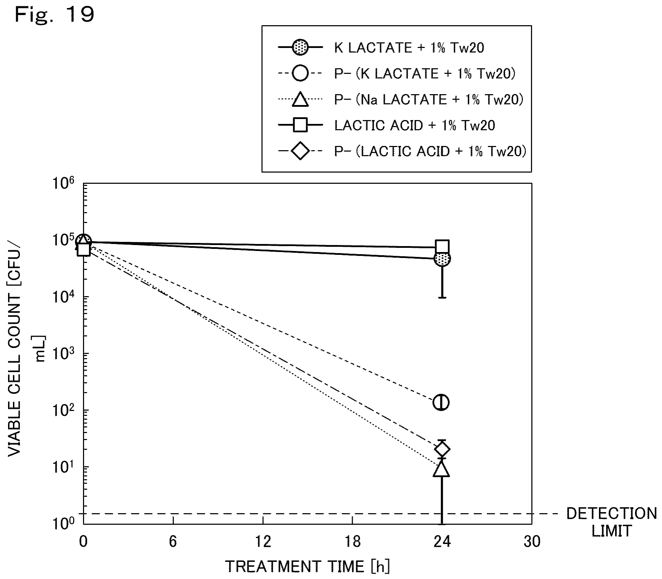

Fig. 19 shows the results of an experiment on sterilization of fungal (Penicillium digitatum) spores by a plasma-irradiated aqueous solution obtained by irradiating an aqueous solution containing lactic acid or potassium lactate and a surfactant with plasma.

As shown in Fig. 19, it was confirmed that even when lactate (potassium lactate) or lactic acid was used instead of sodium lactate, by mixing it with a surfactant and irradiating this aqueous solution with plasma, a sterilization effect against true fungi or fungi was exhibited, similar to the case of using sodium lactate.

Note that, in this experimental example, no experiment was performed using calcium lactate, which is an alkaline earth metal lactate, but it can be understood that calcium lactate has similar properties in terms of being alkaline as compared to sodium lactate and potassium lactate, which are alkali metal lactate. Therefore, even when a plasma-irradiated aqueous solution obtained by irradiating an aqueous solution containing calcium lactate and a surfactant with plasma is used, it can be understood that a sterilization effect against true fungi or fungi can be exhibited, similar to when a plasma-irradiated aqueous solution obtained by irradiating an aqueous solution containing sodium lactate, potassium lactate, or lactic acid and a surfactant with plasma is used.

The present disclosure is not limited to the above-described embodiments and experimental examples, and various modifications can be made without departing from the gist of the present disclosure. Further, the configurations shown in each embodiment and each experimental example can be arbitrarily combined with each other. Further, the claims described in the scope of claims as originally filed can be arbitrarily combined with each other.

Note that, since the plasma-irradiated aqueous solution in the present disclosure has a specific matter of "obtained by irradiating an aqueous solution with plasma", there is a possibility that it is identified as being unclear because an object is specified by a description of a manufacturing method. However, in order to newly find a difference between substances in both aqueous solutions of an aqueous solution not irradiated with plasma and an aqueous solution after being irradiated with plasma, characteristics and indicators for distinguishing both aqueous solutions, and the like, it is necessary to perform many additional trials and errors. Furthermore, if conditions such as the type of surfactant are different, the types of specific substances contained after plasma irradiation may also be different. Therefore, it is necessary to perform an unrealistic number of times of experiments, analyses, evaluations, and the like for each of them, which would result in enormous time and cost. Therefore, regarding the plasma-irradiated aqueous solution of the present disclosure including the above-described specific matters, it must be said that "directly specifying the object by its structure or characteristics at the time of filing" is not practical. Therefore, it can be said that the plasma-irradiated aqueous solution of the present disclosure is clear. The same can be said for the plasma-irradiated aqueous solution generation device.

## Claims

1. A plasma-irradiated aqueous solution obtained by irradiating an aqueous solution containing at least one selected from the group consisting of sodium lactate, potassium lactate, calcium lactate, and lactic acid, and a surfactant, with plasma.

2. The plasma-irradiated aqueous solution according to claim 1, wherein the surfactant is at least one selected from a nonionic surfactant, a cationic surfactant, an anionic surfactant, and an amphoteric surfactant.

3. The plasma-irradiated aqueous solution according to claim 1 or 2, wherein the aqueous solution contains at least the sodium lactate.

4. The plasma-irradiated aqueous solution according to claim 1 or 2, wherein a concentration of the surfactant in the aqueous solution is 2% by volume or less.

5. A method for producing a plasma-irradiated aqueous solution, comprising:
a preparation step of preparing an aqueous solution containing at least one selected from the group consisting of sodium lactate, potassium lactate, calcium lactate, and lactic acid, and a surfactant; and
a plasma irradiation step of irradiating the aqueous solution prepared in the preparation step with plasma to generate a plasma-irradiated aqueous solution.

6. The method for producing a plasma-irradiated aqueous solution according to claim 5, wherein the surfactant is at least one selected from a nonionic surfactant, a cationic surfactant, an anionic surfactant, and an amphoteric surfactant.

7. The method for producing a plasma-irradiated aqueous solution according to claim 5 or 6, wherein the aqueous solution contains at least the sodium lactate.

8. The method for producing a plasma-irradiated aqueous solution according to claim 5 or 6, wherein a concentration of the surfactant in the aqueous solution is 2% by volume or less.

9. A plasma-irradiated aqueous solution generation device having a plasma irradiation unit configured to irradiate an aqueous solution containing at least one selected from the group consisting of sodium lactate, potassium lactate, calcium lactate, and lactic acid, and a surfactant, with plasma to generate a plasma-irradiated aqueous solution.

10. A sterilization method in which the plasma-irradiated aqueous solution according to claim 1 or 2, a plasma-irradiated aqueous solution obtained by the method for producing a plasma-irradiated aqueous solution according to claim 5 or 6, or a plasma-irradiated aqueous solution generated by the plasma-irradiated aqueous solution generation device according to claim 9 is brought into contact with true fungi or fungi to sterilize the true fungi or the fungi.
